# EUROPEAN PATENT SPECIFICATION

(11) **EP 4 670 027 B1**
(45) Date of publication and mention of the grant of the patent: **28.10.2026**
(21) Application number: 24704837.4
(22) Date of filing: 15.02.2024
(51) Int. Cl.: G06F 3/01, G06F 3/04815, G06T 19/00, A61B 5/00, A61B 5/11

(54) **AUGMENTED REALITY RENDERING METHOD**
RENDERINGVERFAHREN FÜR ERWEITERTE REALITÄT
PROCÉDÉ DE RENDU DE RÉALITÉ AUGMENTÉE

(30) Priority: 24.02.2023 NL 2034220
(43) Date of publication of application: 31.12.2025
(73) Proprietor: LAGENDIJK, Theodoor Simon, 3027 TM Rotterdam (NL)
(72) Inventor: LAGENDIJK, Theodoor Simon, 3027 TM Rotterdam (NL)
(74) Representative: Arnold & Siedsma
(86) International application number: PCT/EP2024/053928
(87) International publication number: WO 2024/175474

(56) References cited:
- WO-A1-2016/164342
- US-A1- 2020 394 012

## Description

The present invention relates to an augmented reality rendering method, and associated devices and system, for remote assistance of a user by an expert in performing a task in a work environment.

Typically, in the field of automotive maintenance/service/repair, operatives/users face the need of an assistance in performing a task in their environment. Yet many other fields in which a user may require the assistance of an expert demonstrating how to perform a task may be envisaged. Whenever a local expert is not available, remote assistance may be of interest.

Typically, remote assistance is provided via electronic means. A user may hold an electronic device while an expert may hold another electronic device, both devices being configured for communication with each other over standard communication networks. Augmented reality interfaces on such electronic devices have also been used in such context to connect a user and an expert.

Known methods of augmented reality rendering for remote assistance have yet been limited to video/image sharing on which virtual markers may be added. The prior art known solutions, such as known from the patent applications: US2020/394012 A1 and WO2016/164342 A1, do not yet allow for rendering complex manipulations. In particular known methods do not allow for an expert to easily demonstrate manual manipulations to be performed by the user in his own work environment.

An object of the invention, next to other objects, is to provide an improved augmented reality rendering method solving the drawbacks of the prior art.

This object, next to other objects, is met by an augmented reality rendering method for remote assistance of a user in performing a task in a work environment. The assistance being provided via electronic means by an expert, said electronic means comprising a user device associated with the user and an expert device associated with the expert, the user device and the expert device being configured for communicating with each other, the method comprising the steps of obtaining user scene information from the user device, the user scene information comprising image data of the work environment, displaying the user scene information on the expert device, obtaining expert scene information from the expert device, the expert scene information comprising image data of at least an object controllable by the expert for showing how to perform the task, creating a model of the at least one controllable object based on the obtained expert scene information, generating an augmented reality AR scene by including the created model of the at least one controllable object in the image data of the work environment, displaying the augmented reality AR scene on the expert device and on the user device.

In this way, the expert may easily demonstrate in the augmented reality scene the movements of the object to perform the task while looking himself at the work environment seen by the user in reality, and thus as if sharing the same reality as the user. An intuitive yet detailed rendering of complex object movements is thus achieved by the claimed method. The work environment as seen by the user device, i.e. from the perspective of the user device, may be called the user scene.

Preferably, the model is a model of the object seen from the perspective of the expert. In this way, the perspectives of the expert and the user are aligned, achieving thus an efficient assistance of the user. Since the image data, preferably real-time video data, of the expert controllable object is captured by the expert camera from a point of view opposite to the point of view of the expert, artificially changing this perspective is part of the process of constructing a model of the expert controllable object.

Preferably, obtaining expert scene information comprises collecting expert scene image data from at least a camera of the expert device, the at least one camera facing the expert, wherein the expert scene image data is preferably real-time video data of the expert scene. In this way, the expert may look at the user scene information on the display while the camera may obtain the expert scene including the object in between the expert and the display. In other words, the orientation of the camera and of the display allow for the object to be located in between the expert and the display, so the expert can naturally superimpose his view of the object onto the displayed information. Using real-time video data may further provide the benefits of typical video conferencing for a more efficient user/expert communication.

Preferably, collecting image data from at least a camera of the expert device comprises collecting image data using a single camera, said camera being arranged on the same side of the expert device as the display and preferably in close proximity thereof. In this way, a simple expert device with a single front camera next to the display may be used, such as a tablet with a front facing "selfie" camera, or a laptop with a webcam. By close proximity is meant in the same, typically top central, portion of the electronic device. By sharing a line of view between the camera and the display, the step of creating a model may be simplified by assuming the position of the expert with respect to the display/camera. Alternatively, the camera may be an external camera oriented towards the expert with a different line of view to the expert than the line of view of the expert towards the display.

Preferably, creating a model of the at least one controllable object based on the obtained expert scene information comprises extracting, from the image data of the at least one object, data on the at least one object seen from the perspective of the expert device camera, and estimating the model of the at least one object seen from the expert perspective based on the extracted data.

In this way, a model of the controllable object as seen from the expert perspective is obtained from the image obtained by the device camera. By expert perspective is meant the viewing perspective from the expert eyes. By perspective of the expert device camera is meant the observing perspective from the camera In other words, the perspective of the controllable object as observed by the camera is rotated 180 degrees to derive a perspective of the model of the object. This 180 degrees rotated (also called reversed later on) perspective amounts to the expert perspective on the object, i.e. how the expert views the object from his own eyes.

Preferably, the at least one object controllable by the expert comprises any one or more of the following: a body part of the expert, in particular at least one hand of the expert, a tool manipulated by the expert, a device controlled by the expert. In this way, the expert can demonstrate actions to be performed by the user by using for example his hands, a tool or another device. This gives an intuitive method by which to demonstrate how the user should perform actions. The expert can for example use his hands to point out relevant parts of the work environment, or to show how to manipulate a relevant part.

Preferably, image data of the work environment comprises still image or real-time video data of the work environment. In this way two options are provided to the user and the expert. In some cases using real-time video data could be most preferred, as it allows the user to perform actions as instructed, and the expert to see the actions being performed and their effects. However, the camera on the user device should be kept as still as possible, because otherwise the expert has to continually adjust the positioning of for instance his hands as a consequence of the user scene continually changing. The user device can be kept still by, for example, setting the user device on a steady surface, or mounting it on a tripod or similar device. If this is not possible, it might be more preferred to provide a still image of the user scene to the expert, so as to prevent this user scene from continually changing.

Preferably, user scene information further comprises image data of the user, preferably real-time video data of the user. In this way, communication between the user and the expert is improved, because in addition to the expert seeing the user scene, the expert also directly sees the user. The functionalities of typical video conferencing may thus be integrated in the method for a more efficient user/expert communication. The image data of the user may be obtained from a camera of the user device or an external additional camera communicating with the user and/or expert device.

Preferably, displaying the user scene information on the expert device comprises displaying in a first window image data of the work environment. In this way, the user scene is displayed in a distinct, preferably as large as possible given the display size, window.

Preferably, displaying the user scene information on the expert device comprises displaying in a second window image data of the user. In this way image data of the user is displayed to the expert while intruding on the display space available for the user scene as little as possible. The second window may thus operate as a video conferencing window for a more efficient user/expert communication.

Preferably, obtaining user scene information from the user device further comprises obtaining sound data of the work environment and/or of the user, and wherein obtaining expert scene information from the expert device comprises obtaining sound data of the expert, the method further comprising outputting the sound data of the expert to the user and the sound data of the user to the expert. In this way the user and expert can hear each other and can therefore converse with each other. In addition, the sound data may comprise sounds picked up from the work environment which can further inform the expert on a condition of the work environment, for instance a condition of operation of an apparatus in said environment.

Preferably, creating a model of the at least one controllable object comprises creating a 2D model of the at least one controllable object. In this way, a simplified model of the controllable object can be made. This model can then be included in the augmented reality scene on the user device. A 2D representation of the controllable object takes up little space in the user scene while providing sufficient precision. As a consequence, the representation of the controllable object in the augmented reality scene is barely intrusive. The 2D model being non-intrusive, loosing visual information is thus prevented and the accuracy in showing the task is achieved. In addition, creating a 2D model requires less computing resources and also less computing time which is advantageous in the context of real-time interaction. If the controllable object comprises one or more hands, these hands may be schematically represented by a meshed outline of the palm and the fingers. If the controllable object comprises a tool or a device, the 2D representation may comprise an outline of the tool or device, which is typically sufficient to interpret the intention of the expert. Alternatively, a 3D model may be created, for instance a point cloud model or a volumetric mesh model.

Preferably, creating a 2D model of the at least one controllable object comprises identifying characteristic points, preferably joints, of the controllable object, and creating a 2D outline representation of the controllable object based on the identified characteristic points, preferably joints. In this way, a simple but pertinent 2D model is obtained, showing a minimal outline for a precise but not intrusive representation of the controllable object. Alternatively, a 3D model may be created of hands or static objects (not changing shapes).

Another aspect relates to a storage medium for storing instructions of a program, which when executed on a processor causes the steps of any of the above method embodiments to be performed. This allows for these instructions, alternatively referred to as "software" for simplicity, to be loaded on a variety of common electronic devices such as phones, tablets and laptops.

Another aspect relates to an electronic user device for remote assistance of a user by an expert for performing a task in a work environment, said user device being associated with the user, and comprising at least a camera for obtaining user scene information, the user scene information comprising image data of the work environment, communication means for communicating the user scene information to the expert device and for receiving an augmented reality AR scene from the expert device, a display for displaying the received augmented reality AR scene, a processor configured to perform one or more steps of any of the above method claims. In this way, all the required components for performing the actions related to the user of the method according to any of the preceding embodiments are contained in a single device that can be used by the user.

Preferably, the processor of the user device is configured to display the augmented reality AR scene in a first window of the display.

Preferably, the processor of the user device is configured to display image data of the expert in a second window of the display. More preferably the second window is a relatively smaller window within the first window. In this way, the augmented reality scene can be displayed in a main window on the user device, and the expert scene in a relatively smaller insert window to maximize the display space used for the user scene and the augmented reality scene.

Preferably the electronic user device is any of the following: a tablet, a smartphone, a laptop, one or more head mounted displays, for instance googles. In this way, the aforementioned method can be employed on readily available, unmodified, consumer electronic devices.

Another aspect relates to an electronic expert device for remote assistance of a user by an expert for performing a task in a work environment , said expert device being associated with the expert, and comprising at least one camera, wherein the camera is configured for obtaining expert scene information, the expert scene information comprising image data at least an object controllable by the expert for showing how to perform the task, a processor configured to perform one or more steps of any of the above method claims, and at least configured for a) creating a model of the at least one controllable object from the obtained expert scene information, b) generating an augmented reality AR scene by including the created model of the at least one controllable object, communications means for receiving a user scene information from the user device, and for communicating to the user device the augmented reality AR scene, a display arranged, for displaying the user scene information on the expert device, and the augmented reality AR scene. In this way, all the required components for performing the actions related to the expert of the method according to any of the preceding embodiments are contained in a single expert device.

Preferably, the camera and the display are arranged on the same side of the expert device, and preferably in close proximity.

Preferably, the device comprises a single camera. In this way, a device with only a front camera is sufficient.

Preferably, the processor of the expert device is configured to display the augmented reality AR scene in a first window of the display.

Preferably, the processor of the expert device is configured to display image data of the user in a second window of the display. More preferably the second window is a relatively smaller window within the first window. In this way the augmented reality scene can be displayed in a main window on the expert device, and the image data of the user in a relatively smaller insert window to maximize the display space used for the user scene and the augmented reality scene.

The electronic expert device of any of the aforementioned embodiments, being any of the following: a tablet, a smartphone, a laptop. In this way, the aforementioned method can be employed on readily available, unmodified, consumer electronic devices.

Another aspect relates to an assistance system comprising an electronic user device according to any of the aforementioned preferred embodiments and an electronic expert device according to any of the aforementioned preferred embodiments.

This and other aspects of the present invention will now be described in more detail, with reference to the appended drawings showing currently preferred embodiments of the invention, wherein:
- Figure 1 illustrates a schematic representation of an assistance system according to an embodiment of the invention;
- Figure 2 illustrates a schematic view of an electronic user device in use according to an embodiment of the invention;
- Figure 3 illustrates a schematic view of an electronic expert device according to an embodiment of the invention
- Figure 4 illustrates a schematic view of an electronic expert device during remote assistance according to an embodiment of the invention;
- Figure 5 illustrates the display of an electronic expert device during remote assistance according to an embodiment of the invention;
- Figure 6 illustrates the display of an electronic user device during remote assistance according to an embodiment of the invention;
- Figure 7 illustrates a schematic representation of an electronic user/ expert device according to embodiments of the invention;
- Figure 8 illustrates a flowchart of the steps of a method according to an embodiment of the invention.

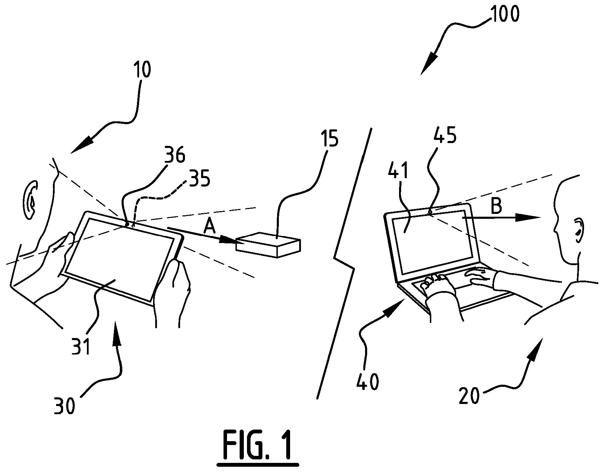

Figure 1 shows a schematic representation of an assistance system 100 according to an embodiment of the invention. The assistance system 100 is for remotely assisting a user 10 in performing a task in a work environment 15. The user 10 is located in a location where he can look at and interact with the work environment 15 while the expert 20 providing the assistance and demonstrating the manipulations to be performed in the work environment 15 is located in a remote location. The task may comprise one or more, for example manual, manipulations that need to be performed by the user 10. The task may comprise among others installing/repairing/configuring one or more apparatuses present in the work environment 15. The assistance is provided via electronic means comprising an electronic user device 30 used by the user 10, and an electronic expert device 40 used by the expert 20. The electronic user device 30 may be further referred to in the rest of the text simply as a user device. Similarly, the electronic expert device 40 may be further referred to in the rest of the text simply as an expert device. The user device 30 and the expert device 40 are further configured to communicate with each other, typically over at least a wireless network.

The user device 30 may comprise a display 31, a first user camera 35 and optionally a second user camera 36. The user device 30 may be, for example, a tablet, a phone or a laptop comprising a rear camera and a front camera as first and second user cameras 35 and 36 respectively. The first user camera 35 may be oriented in a direction A opposite to the display direction of the display 31, and optionally opposite to the direction of the second user camera, so that when the user 10 orients the first camera 35 towards the work environment 15, the display 31, and optionally the second user camera 36, may be oriented towards the user 10.

Similarly, the expert device 40 may comprises a display 41 and an expert camera 45. The expert camera 45 may be oriented towards the expert 20 in a direction B to capture an expert scene. By expert scene is meant a scene containing the expert, typically his hands and face, seen from the expert camera 45. The expert camera 45 may be configured to obtain expert scene image data 61 of the expert scene, more preferably real time video of the expert scene. The expert device 40 may be, for example, a laptop comprising a front camera as the expert camera 45.

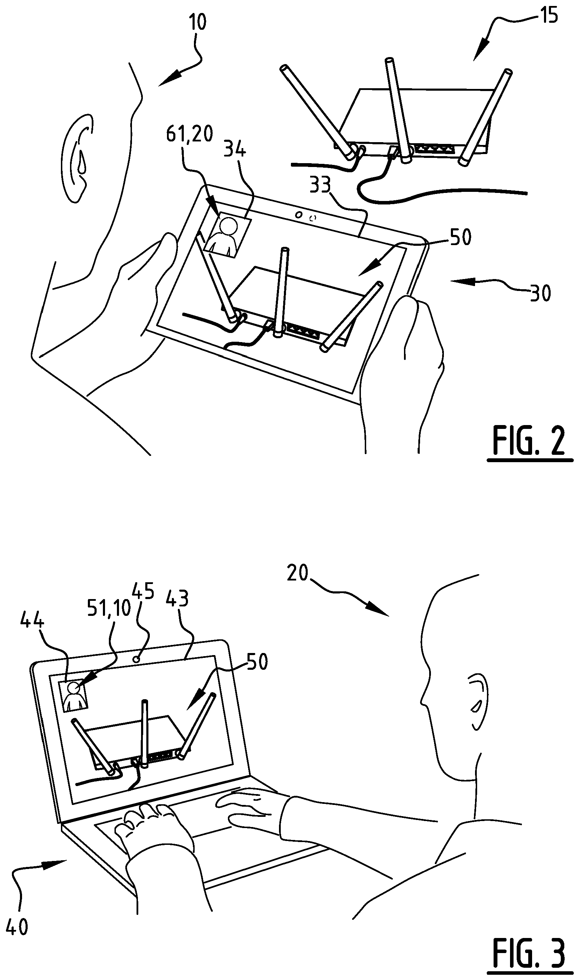

Figures 2 and 3 show a user device 30 and an associated expert device 40 according to an embodiment. The user device 30 and expert device 40 may be used in the shown figures for connecting a user 10 and an expert 20 such that the expert 20 may assist the user 10 in installing a Wifi-router in the work environment 15. The first user camera 35 may obtain user scene information 50, and the optional second user camera 36 may obtain image data 51 of the user 10, preferably of the user's face. By user scene information is meant information retrieved at the location of the user without specifying any perspective of viewing. User scene information may comprise image data 50 of the work environment 15 (said image data 50 of the work environment 15 being also called simply user scene 50 in the rest of the text) and optionally image data 51 of the user 10. The first user camera 35 may obtain the image data 50, preferably real-time video, of the work environment 15. This image data 50 may be displayed in a main user window 33 on the user display 36. In this way, while looking at the display 36, the user 10 may move the user device 30 such as to obtain a clear view on the display 31 of the work environment 15 in which the task in to be performed. In the case of Figure 2, the user 10 may move the user device 30 to have a clear view of the Wifi router. In addition, a secondary user window 34 may be displayed on user display 36. Inside this secondary user window 34, image data 61 of the expert 20 from the expert device 40 may be displayed. The image of the expert 20 may be real-time video data of the expert.

The expert device 40 may receive the user scene information from the user device 30 of Figure 2. The received user scene information may then be displayed on the expert device 40. Image data 50 of the work environment 15 may be displayed inside a main expert window 43. Image data 51 of the user 10 may be displayed inside a secondary expert window 44. As discussed above, the expert camera 45 may be oriented towards the expert 20 and may obtain expert scene information, comprising image data 61 of the expert scene and optionally sound data of the expert scene. In the show example, the hands of the expert are outside of view field of the camera 45, such that the expert scene image data 61 may only capture the face and upper body of the expert 20. This can be seen inside window 34 of the user device 30 of Figure 3.

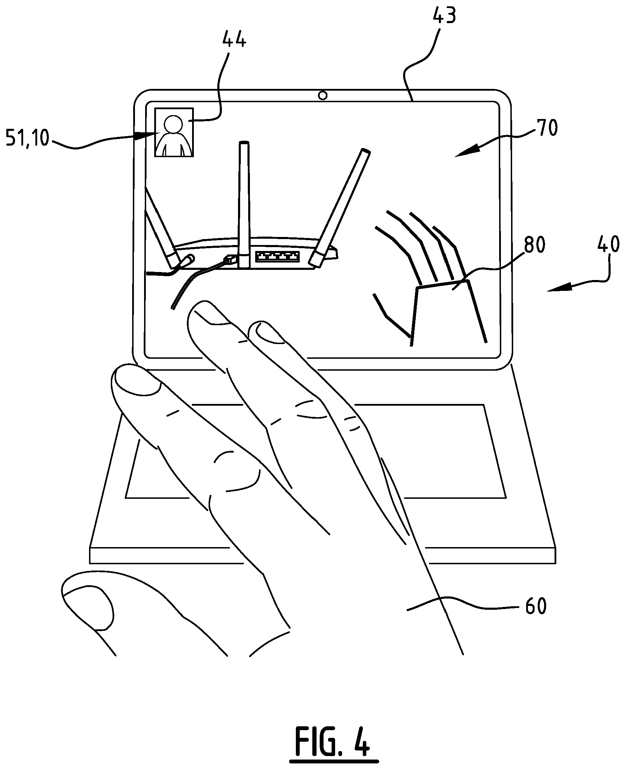

Figure 4 shows a schematic view of an electronic expert device 30 in use during remote assistance. In contrast to Figure 3, the expert 20 during remote assistance may place at least an object 60 in between his eyes and the expert device 40. In Figure 4, the expert simply uses his hands as object 60, and raises them in front of the user device so that they may come into the view field of the expert camera 45. Although hands are a preferred object 60 for showing how to perform the task, the teachings of the application apply to an object in general including a tool, and/ or a device that may also be added or used instead of hands. The expert scene information during remote assistance comprises image data of the object 60 controllable by the expert 20. As previously disclosed in Figure 3, image data 50 of the work environment 15 is displayed in the expert window 43. The expert 20 sees this work environment 15 comprising in the given example a WiFi Router, and moves his hand(s) to indicate areas of interest and explain to the user 10 how to, for example, perform certain operations in the work environment 15. The expert scene image data 61 comprising the hands may then be used to create a model 80 of the hands. A software may perform this model creation steps. Such a software may recognize the hands 60, and generates a simplified 2D representation/model 80, also called here further outline, of the hands. This outline 80 may then be overlaid (another word would be superposed) on the user scene 50, thereby creating an Augmented Reality (AR) scene 70 which is simultaneously displayed on the user device 30 and expert device 40. Generating the AR scene 70 may take place in real-time, such that movements of the hands may be displayed in the AR scene 70 with minimal delay. The relative positioning of the outline 80 with respect to the user scene 50 is intuitively adapted by the expert 20 by moving his hands such that the outline 80 matches the position on the user scene 50 which the expert intends to reach.

The model/outline 80 may be created using image data from the expert camera 45 having a viewing perspective along the direction B of Figure 1 which is 180 degrees rotated with respect to the perspective of the expert. This may be achieved in two steps.

First data regarding the hands (more generally the object) may be extracted from the expert scene image data 61 to generate a first 2D representation of the hands using a first computer implemented method/software. The first software may for instance be a known software offering a high-fidelity hand and finger tracking solution. The first steps of extracting data regarding the hands may comprise identifying joints (or characteristic points/landmarks of the object in general) of the hands and connecting them to create a 2D outline representation of the hands. It may employ machine learning (ML) to infer 3D landmarks (characteristic points) of a hand from just a single frame, and it may optionally process the latest frame together with information from the preceding frame(s) in order to increase processing efficiency. It may consist of multiple models working together. A palm detection model may operate on the full image and return an oriented hand bounding box. A hand landmark model may operate on the cropped image region defined by the palm detector and may return high fidelity 3D hand keypoints. Providing the accurately cropped hand image to the hand landmark model may reduce the need for data augmentation (rotations, translation and scale) and allow the network to dedicate most of its capacity towards coordinate prediction accuracy. This first extracted 3D representation of the hands may then be a view of the hands as seen from the expert camera 45.

Using a second computer implemented method/software based on pose estimation, this extracted 3D representation may then be rotated to match the expert perspective. For example, in many cases some parts of the hand may obstruct other parts of the hand from the view of the expert camera 45. The first software may recognize this and compensate for the obstruction when creating a 3D representation of the hand. When the 3D representation of the hand is then rotated to match the expert perspective, the obstructed parts may be made visible to the expert and the user. The 3D perspective of the hand may additionally be indicated in the 2D representation of the hand by means of one or more of colour, transparency, line thickness.

The second step of reversing the perspective may be achieved because the relative position of the eyes of the expert 20 with respect to the expert camera 45 may be largely assumed to be known. Any accuracy in the estimation of the expert's location with respect to the camera may be later intuitively corrected by the expert 20 self by moving his hands in front of the camera 45. This solution is in that sense simple and robust.

Finally, the reversed 3D model from the expert's perspective may be simplified into the 2D model 80, before being included into the augmented reality scene 70.

It is noted that although preferably the creation of the model may be performed on the expert device 30, the operation could equally be performed in a remote server in communication with both devices, or in the user device 30.

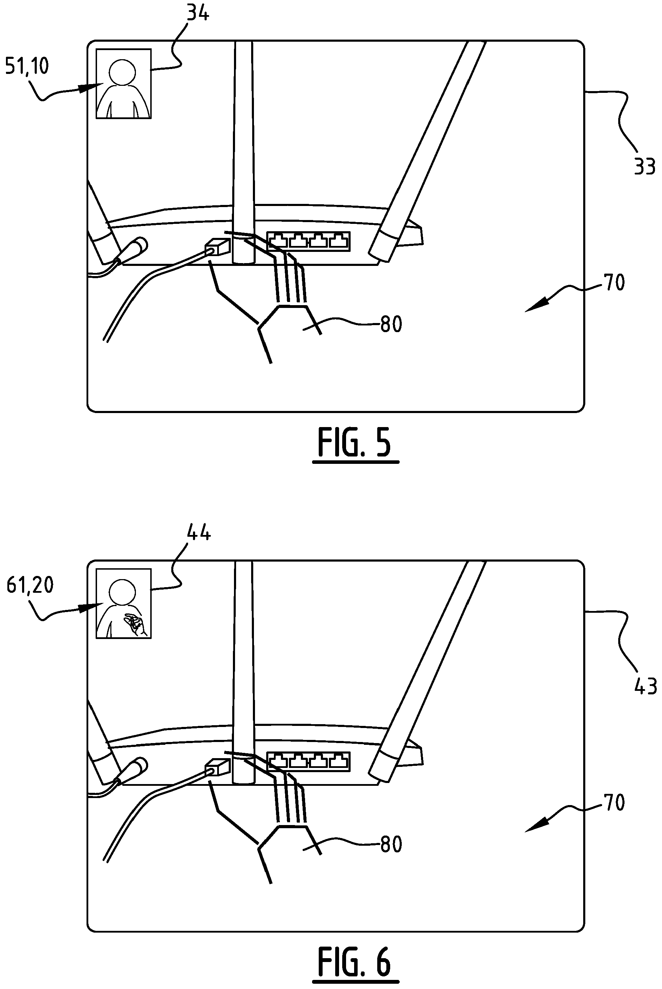

Figures 5 and 6 show what is displayed on user display 33 and expert display 43 at the same point in time during remote assistance. The same AR scene 70 containing the 2D representation 80 of the hands 60 overlaid on the user scene 50 may be simultaneously (as simultaneous as possible given generally expectable slight processing and transmission delays) displayed on the user display 31 and expert display 41, preferably in the dedicated mains windows 33 and 43. In addition, in secondary expert window 44 image data, preferably real time video data, of the user 10 may be displayed. In secondary user window 34 image data 61, preferably real time video data, of the expert scene comprising the expert 20 is displayed.

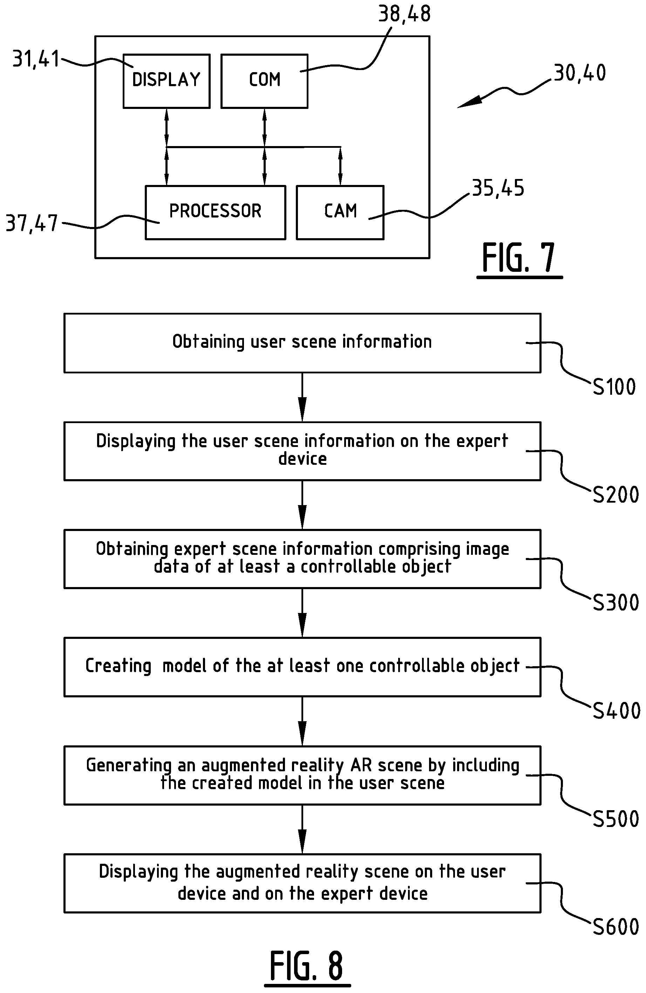

Figure 7 shows a schematic representation of the inner construction of an electronic user/expert device 30 or 40. The devices 30, 40 may comprise among others respectively a camera 35/45 (and additional camera 36 not represented for device 30), a display 31/41, telecommunication means 38/48, and a central processor 37/47. All above mentioned components are connected to a central data bus for internal communication. Additional memories, interfaces, sensors may of course be further available depending on the circumstances and as known in the art.

Figure 8 shows a schematic representation of the method for remote assistance according to the invention. Some of the steps of said methods may be computer implemented. For that purpose, the devices 30 and 40 comprises respectively the processors 37 and 47 and memories (not represented) for storing instructions which when executed on the processor cause the steps of the method to be performed. In the first step S100, user scene 50, preferably real time video data, is obtained. This user scene 50 is then displayed on the user expert device 40 in the second step S200. Subsequently in step S300 expert scene image data 61, preferably real time video comprising the expert 20 and expert device 60 is generated by expert camera 45. In step S400, a model of the at least one controllable object is created. The next step S500 comprises generating an augmented reality AR scene by including the creates model in the user scene 50. In step S600 the AR scene 70 is then displayed on the expert device 40 and on the user device 30.

Whilst the principles of the invention have been set out above in connection with specific embodiments, it is understood that this description is merely made by way of example and not as a limitation of the scope of protection which is determined by the appended claims.

## Claims

1. Augmented reality rendering method for remote assistance of a user (10) in performing a task in a work environment (15), the assistance being provided via electronic means (30, 40) by an expert (20), said electronics means (30, 40) comprising a user device (30) associated with the user (10) and an expert device (40) associated with the expert (20), the user device (30) and the expert device (40) being configured for communicating with each other, the method comprising the steps of:
- obtaining (100) user scene information from the user device (20), the user scene information comprising image data (50) of the work environment (15),
- displaying (200) the user scene information on the expert device (40),
- obtaining (300) expert scene information from the expert device (40), the expert scene information comprising image data of at least an object (60) controllable by the expert (20) for showing how to perform the task,
- creating (400) a model (80) of the at least one controllable object (60) based on the obtained expert scene information,
- generating (500) an augmented reality AR scene (70) by including the created model (80) of the at least one controllable object (60) in the image data (50) of the work environment (15),
- displaying (600) the augmented reality AR scene (70) on the expert device (40) and on the user device (30),
wherein the model (80) is a model of the object (60) seen from the perspective of the expert (20), wherein obtaining (300) expert scene information comprises collecting expert scene image data (61) from at least a camera (45) of the expert device (40), the at least one camera facing the expert (20) such that the perspective of the expert (20) is opposite the perspective of the camera (45) of the expert device (40).

2. The method of claim 1, wherein the expert scene image data is real-time video data of the expert scene, and/or wherein collecting image data from at least a camera (45) of the expert device (40) comprises collecting image data using a single camera (45), said camera being arranged on the same side of the expert device (40) as the display (41) and preferably in close proximity thereof.

3. The method of claim 2, wherein creating (400) a model of the at least one controllable object (60) based on the obtained expert scene information comprises:
- extracting, from the image data of the at least one object (60), data on the at least one object (60) seen from the perspective of the expert device camera (45), and
- estimating the model of the at least one object seen from the expert perspective based on the extracted data.

4. The method of any of the above claims, wherein the at least one object (60) controllable by the expert (20) comprises any one or more of the following: a body part of the expert (20), in particular at least one hand of the expert, a tool manipulated by the expert (20), a device controlled by the expert (20).

5. The method of any of the above claims, wherein image data of the work environment (15) comprises still image or real-time video data of the work environment (15, and/or wherein user scene information further comprises image data of the user (10), preferably real-time video data of the user (10).

6. The method of any of the above claims, wherein displaying (200) the user scene information on the expert device (40) comprises displaying in a first window (43) image data of the work environment (15), wherein preferably displaying (200) the user scene information on the expert device (40) comprises displaying in a second window (44) image data of the user (10).

7. The method of any of the above claims, wherein obtaining (100) user scene information from the user device further comprises obtaining sound data of the work environment (15) and/or of the user (10), and wherein obtaining (300) expert scene information from the expert device (40) comprises obtaining sound data of the expert (20), the method further comprising outputting the sound data of the expert (20) to the user (10) and the sound data of the user (10) to the expert (20) .

8. The method of any of the above claims, wherein creating (400) a model of the at least one controllable object (60) comprises creating a 2D model (80) of the at least one controllable object (60) , and/or wherein creating (400) a 2D model of the at least one controllable object comprises identifying characteristic points, preferably joints, of the controllable object, and creating a 2D outline representation of the controllable object based on the identified characteristic points, preferably joints.

9. Storage medium for storing instructions of a program, which when executed on a processor causes the steps of any of the above method claims to be performed.

10. Electronic user device (30) for remote assistance of a user (10) by an expert (20) having an expert device (40) for performing a task in a work environment (15), said user device (30) being associated with the user (10), and comprising:
- at least a camera (35) for obtaining image data of the work environment (15),
- communication means (36) for communicating the image data (50) of the work environment (15) to the expert device (40) and for receiving an augmented reality AR scene (70) from the expert device (40),
- a display (31) for displaying the received augmented reality AR scene (70),
- a processor (37) configured to perform at least one or more steps of any of the above method claims.

11. The electronic user device of the previous claim, wherein the processor (37) is configured to display the augmented reality AR scene (70) in a first window (33) of the display (31), wherein preferably the processor (37) is configured to display image data of the expert (20) in a second window (34) of the display, and/or the device being any of the following: a tablet, a smartphone, a laptop, one or more head mounted displays.

12. Electronic expert device (40) for remote assistance of a user (10) having a user device (30) by an expert (20) for performing a task in a work environment (15), said expert device being associated with the expert, and comprising
- at least one camera (45), wherein the camera (45) is configured for obtaining expert scene information, the expert scene information comprising image data at least an object controllable by the expert for showing how to perform the task,
- a processor configured to perform at least one or more steps of any of the above method claims, and at least configured for:
creating a model of the at least one controllable object from the obtained expert scene information,
generating an augmented reality AR scene by including the created model of the at least one controllable object in image data of the work environment (15),
- communications means (46) for receiving a user scene information from the user device comprising image data of the work environment (15), and for communicating to the user device the augmented reality AR scene,
- a display (41) arranged, for displaying the user scene information on the expert device, and the augmented reality AR scene.

13. The electronic expert device of the previous claim, wherein the camera and the display are arranged on the same side of the expert device, and preferably in close proximity, and/or wherein the device comprises a single camera.

14. The electronic expert device of any of claims 12-13, wherein the processor (47) is configured to display the augmented reality AR scene (70) in a first window (43) of the display (41), wherein preferably the processor (37) is configured to display image data of the user (10) in a second window (44) of the display (41), and/or the device being any of the following : a tablet, a smartphone, a laptop.

15. An assistance system comprising an electronic user device according to any of claims 10-11 and an electronic expert device according to any of claims 12-14.

## Patentansprüche

1. Augmented-Reality-Rendering-Verfahren für eine Fernunterstützung eines Benutzers (10) bei einem Durchführen einer Aufgabe in einer Arbeitsumgebung (15), wobei die Unterstützung über elektronische Mittel (30, 40) durch einen Experten (20) bereitgestellt wird, die elektronischen Mittel (30, 40) umfassend eine Benutzervorrichtung (30), die dem Benutzer (10) zugeordnet ist, und eine Expertenvorrichtung (40), die dem Experten (20) zugeordnet ist, wobei die Benutzervorrichtung (30) und die Expertenvorrichtung (40) zum Kommunizieren miteinander konfiguriert sind, das Verfahren umfassend die Schritte:
- Erhalten (100) von Benutzerszeneninformationen von der Benutzervorrichtung (20), die Benutzerszeneninformationen umfassend Bilddaten (50) der Arbeitsumgebung (15),
- Anzeigen (200) der Benutzerszeneninformationen auf der Expertenvorrichtung (40),
- Erhalten (300) von Expertenszeneninformationen von der Expertenvorrichtung (40), die Expertenszeneninformationen umfassend Bilddaten von mindestens einem Objekt (60), das durch den Experten (20) steuerbar ist, zum Vorführen, wie die Aufgabe durchzuführen ist,
- Erstellen (400) eines Modells (80) des mindestens einen steuerbaren Objekts (60) basierend auf den erhaltenen Expertenszeneninformationen,
- Erzeugen (500) einer Augmented-Reality-Szene, AR-Szene, (70) durch Einschließen des erstellten Modells (80) des mindestens einen steuerbaren Objekts (60) in die Bilddaten (50) der Arbeitsumgebung (15),
- Anzeigen (600) der Augmented-Reality-Szene, AR-Szene, (70) auf der Expertenvorrichtung (40) und auf der Benutzervorrichtung (30),
wobei das Modell (80) ein Modell des Objekts (60), von der Perspektive des Experten (20) aus gesehen, ist, wobei das Erhalten (300) von Expertenszeneninformationen ein Erfassen von Expertenszenenbilddaten (61) von mindestens einer Kamera (45) der Expertenvorrichtung (40) umfasst, wobei die mindestens eine Kamera dem Experten (20) derart zugewandt ist, dass die Perspektive des Experten (20) der Perspektive der Kamera (45) der Expertenvorrichtung (40) entgegengesetzt ist.

2. Verfahren nach Anspruch 1, wobei die Expertenszenenbilddaten Echtzeitvideodaten der Expertenszene sind, und/oder wobei das Erfassen von Bilddaten von mindestens einer Kamera (45) der Expertenvorrichtung (40) das Erfassen von Bilddaten unter Verwendung einer einzigen Kamera (45) umfasst, wobei die Kamera auf derselben Seite der Expertenvorrichtung (40) wie die Anzeige (41) und vorzugsweise in unmittelbarer Nähe davon angeordnet ist.

3. Verfahren nach Anspruch 2, wobei das Erstellen (400) eines Modells des mindestens einen steuerbaren Objekts (60) basierend auf den erhaltenen Expertenszeneninformationen umfasst:
- Extrahieren, aus den Bilddaten des mindestens einen Objekts (60), von Daten über das mindestens eine Objekt (60), von der Perspektive der Expertenvorrichtungskamera (45) aus gesehen, und
- Schätzen des Modells des mindestens einen Objekts, von der Expertenperspektive aus gesehen, basierend auf den extrahierten Daten.

4. Verfahren nach einem der vorstehenden Ansprüche, wobei das mindestens eine Objekt (60), das durch den Experten (20) steuerbar ist, eines oder mehrere der Folgenden umfasst: ein Körperteil des Experten (20), insbesondere mindestens eine Hand des Experten, ein Werkzeug, das durch den Experten (20) gehandhabt wird, eine Vorrichtung, die durch den Experten (20) gesteuert wird.

5. Verfahren nach einem der vorstehenden Ansprüche, wobei Bilddaten der Arbeitsumgebung (15) Standbild- oder Echtzeitvideodaten der Arbeitsumgebung (15 umfassen und/oder wobei Benutzerszeneninformationen ferner Bilddaten des Benutzers (10), vorzugsweise Echtzeitvideodaten des Benutzers (10), umfassen.

6. Verfahren nach einem der vorstehenden Ansprüche, wobei das Anzeigen (200) der Benutzerszeneninformationen auf der Expertenvorrichtung (40) das Anzeigen von Bilddaten der Arbeitsumgebung (15) in einem ersten Fenster (43) umfasst, wobei das Anzeigen (200) der Benutzerszeneninformationen auf der Expertenvorrichtung (40) vorzugsweise das Anzeigen von Bilddaten des Benutzers (10) in einem zweiten Fenster (44) umfasst.

7. Verfahren nach einem der vorstehenden Ansprüche, wobei das Erhalten (100) von Benutzerszeneninformationen von der Benutzervorrichtung ferner das Erhalten von Tondaten der Arbeitsumgebung (15) und/oder des Benutzers (10) umfasst, und wobei das Erhalten (300) von Expertenszeneninformationen von der Expertenvorrichtung (40) das Erhalten von Tondaten des Experten (20) umfasst, das Verfahren ferner umfassend ein Ausgeben der Tondaten des Experten (20) an den Benutzer (10) und der Tondaten des Benutzers (10) an den Experten (20).

8. Verfahren nach einem der vorstehenden Ansprüche, wobei das Erstellen (400) eines Modells des mindestens einen steuerbaren Objekts (60) das Erstellen eines 2D-Modells (80) des mindestens einen steuerbaren Objekts (60) umfasst, und/oder wobei das Erstellen (400) eines 2D-Modells des mindestens einen steuerbaren Objekts ein Identifizieren von charakteristischen Punkten, vorzugsweise Gliedern, des steuerbaren Objekts und das Erstellen einer 2D-Umrissdarstellung des steuerbaren Objekts basierend auf den identifizierten charakteristischen Punkten, vorzugsweise Gliedern, umfasst.

9. Speichermedium zum Speichern von Anweisungen eines Programms, die, wenn sie auf einem Prozessor ausgeführt werden, verursachen, dass die Schritte nach einem der vorstehenden Verfahrensansprüche durchgeführt werden.

10. Elektronische Benutzervorrichtung (30) für die Fernunterstützung eines Benutzers (10) durch einen Experten (20), der eine Expertenvorrichtung (40) aufweist, zum Durchführen einer Aufgabe in einer Arbeitsumgebung (15), wobei die Benutzervorrichtung (30) dem Benutzer (10) zugeordnet ist und umfasst:
- mindestens eine Kamera (35) zum Erhalten von Bilddaten der Arbeitsumgebung (15),
- Kommunikationsmittel (36) zum Kommunizieren der Bilddaten (50) der Arbeitsumgebung (15) an die Expertenvorrichtung (40) und zum Empfangen einer Augmented-Reality-Szene, AR-Szene, (70) von der Expertenvorrichtung (40),
- eine Anzeige (31) zum Anzeigen der empfangenen Augmented-Reality-Szene, AR-Szene, (70),
- einen Prozessor (37), der konfiguriert ist, um mindestens einen oder mehrere Schritte eines der vorstehenden Verfahrensansprüche durchzuführen.

11. Elektronische Benutzervorrichtung des vorhergehenden Anspruchs, wobei der Prozessor (37) konfiguriert ist, um die Augmented-Reality-Szene, AR-Szene, (70) in einem ersten Fenster (33) der Anzeige (31) anzuzeigen, wobei der Prozessor (37) vorzugsweise konfiguriert ist, um Bilddaten des Experten (20) in einem zweiten Fenster (34) der Anzeige anzuzeigen, und/oder wobei die Vorrichtung ein beliebiges der Folgenden ist: ein Tablet, ein Smartphone, ein Laptop, eine oder mehrere an einem Kopf angebrachte Anzeigen.

12. Elektronische Expertenvorrichtung (40) für die Fernunterstützung eines Benutzers (10), der eine Benutzervorrichtung (30) aufweist, durch einen Experten (20) zum Durchführen einer Aufgabe in einer Arbeitsumgebung (15), wobei die Expertenvorrichtung dem Experten zugeordnet ist und umfasst
- mindestens eine Kamera (45), wobei die Kamera (45) zum Erhalten von Expertenszeneninformationen konfiguriert ist, wobei die Expertenszeneninformationen Bilddaten von mindestens einem Objekt, das durch den Experten steuerbar ist, zum Vorführen, wie die Aufgabe durchzuführen ist, umfassen,
- einen Prozessor, der konfiguriert ist, um mindestens einen oder mehrere Schritte eines der vorstehenden Verfahrensansprüche durchzuführen, und mindestens konfiguriert ist zum:
Erstellen eines Modells des mindestens einen steuerbaren Objekts von der erhaltenen Expertenszeneninformationen,
Erzeugen einer Augmented-Reality-Szene, AR-Szene, durch Einschließen des erstellten Modells des mindestens einen steuerbaren Objekts in Bilddaten der Arbeitsumgebung (15),
- Kommunikationsmittel (46) zum Empfangen von Benutzerszeneninformationen von der Benutzervorrichtung, umfassend Bilddaten der Arbeitsumgebung (15), und zum Kommunizieren der Augmented-Reality-Szene, AR-Szene, an die Benutzervorrichtung,
- eine Anzeige (41), die zum Anzeigen der Benutzerszeneninformationen auf der Expertenvorrichtung und der Augmented-Reality-Szene, AR-Szene, angeordnet ist.

13. Elektronische Expertenvorrichtung des vorhergehenden Anspruchs, wobei die Kamera und die Anzeige auf derselben Seite der Expertenvorrichtung angeordnet sind, und vorzugsweise in unmittelbarer Nähe, und/oder wobei die Vorrichtung eine einzige Kamera umfasst.

14. Elektronische Expertenvorrichtung nach einem der Ansprüche 12 bis 13, wobei der Prozessor (47) konfiguriert ist, um die Augmented-Reality-Szene, AR-Szene, (70) in einem ersten Fenster (43) der Anzeige (41) anzuzeigen, wobei vorzugsweise der Prozessor (37) konfiguriert ist, um Bilddaten des Benutzers (10) in einem zweiten Fenster (44) der Anzeige (41) anzuzeigen, und/oder wobei die Vorrichtung ein beliebiges der Folgenden ist: ein Tablet, ein Smartphone, ein Laptop.

15. Unterstützungssystem, umfassend eine elektronische Benutzervorrichtung nach einem der Ansprüche 10 bis 11 und eine elektronische Expertenvorrichtung nach einem der Ansprüche 12 bis 14.

## Revendications

1. Procédé de rendu de réalité augmentée permettant l'assistance à distance d'un utilisateur (10) pour effectuer une tâche dans un environnement de travail (15), l'assistance étant fournie par l'intermédiaire de moyens électroniques (30, 40) par un expert (20), lesdits moyens électroniques (30, 40) comprenant un dispositif utilisateur (30) associé à l'utilisateur (10) et un dispositif expert (40) associé à l'expert (20), le dispositif utilisateur (30) et le dispositif expert (40) étant configurés pour communiquer l'un avec l'autre, le procédé comprenant les étapes consistant à :
- obtenir (100) des informations de scène utilisateur à partir du dispositif utilisateur (20), les informations de scène utilisateur comprenant des données d'image (50) de l'environnement de travail (15),
- afficher (200) les informations de scène utilisateur sur le dispositif expert (40),
- obtenir (300) des informations de scène expert à partir du dispositif expert (40), les informations de scène expert comprenant des données d'image d'au moins un objet (60) commandable par l'expert (20) pour montrer comment effectuer la tâche,
- créer (400) un modèle (80) de l'au moins un objet commandable (60) sur la base des informations de scène expert obtenues,
- générer (500) une scène de réalité augmentée, RA, (70) en incluant le modèle créé (80) de l'au moins un objet commandable (60) dans les données d'image (50) de l'environnement de travail (15),
- afficher (600) la scène de réalité augmentée, RA, (70) sur le dispositif expert (40) et sur le dispositif utilisateur (30),
dans lequel le modèle (80) est un modèle de l'objet (60) vu de la perspective de l'expert (20), dans lequel l'obtention (300) d'informations de scène expert comprend la collecte de données d'image de scène expert (61) à partir d'au moins une caméra (45) du dispositif expert (40), l'au moins une caméra faisant face à l'expert (20) de telle sorte que la perspective de l'expert (20) soit opposée à la perspective de la caméra (45) du dispositif expert (40).

2. Procédé selon la revendication 1, dans lequel les données d'image de scène expert sont des données vidéo en temps réel de la scène expert, et/ou dans lequel la collecte de données d'image à partir d'au moins une caméra (45) du dispositif expert (40) comprend la collecte de données d'image à l'aide d'une seule caméra (45), ladite caméra étant disposée du même côté du dispositif expert (40) que l'écran (41) et de préférence à proximité immédiate de celui-ci.

3. Procédé selon la revendication 2, dans lequel la création (400) d'un modèle de l'au moins un objet commandable (60) sur la base des informations de scène expert obtenues comprend :
- l'extraction, à partir des données d'image de l'au moins un objet (60), de données sur l'au moins un objet (60) vu de la perspective de la caméra de dispositif expert (45), et
- l'estimation du modèle de l'au moins un objet vu de la perspective de l'expert sur la base des données extraites.

4. Procédé selon l'une quelconque des revendications précédentes, dans lequel l'au moins un objet (60) commandable par l'expert (20) comprend l'un quelconque ou plusieurs parmi : une partie de corps de l'expert (20), en particulier au moins une main de l'expert, un outil manipulé par l'expert (20), un dispositif commandé par l'expert (20).

5. Procédé selon l'une quelconque des revendications précédentes, dans lequel les données d'image de l'environnement de travail (15) comprennent une image fixe ou des données vidéo en temps réel de l'environnement de travail (15), et/ou dans lequel des informations de scène utilisateur comprennent en outre des données d'image de l'utilisateur (10), de préférence des données vidéo en temps réel de l'utilisateur (10).

6. Procédé selon l'une quelconque des revendications précédentes, dans lequel l'affichage (200) des informations de scène utilisateur sur le dispositif expert (40) comprend l'affichage dans une première fenêtre (43) de données d'image de l'environnement de travail (15), dans lequel l'affichage (200) des informations de scène utilisateur sur le dispositif expert (40) comprend de préférence l'affichage dans une seconde fenêtre (44) de données d'image de l'utilisateur (10).

7. Procédé selon l'une quelconque des revendications précédentes, dans lequel l'obtention (100) d'informations de scène utilisateur à partir du dispositif utilisateur comprend en outre l'obtention de données sonores de l'environnement de travail (15) et/ou de l'utilisateur (10), et dans lequel l'obtention (300) d'informations de scène expert à partir du dispositif expert (40) comprend l'obtention de données sonores de l'expert (20), le procédé comprenant en outre la fourniture en sortie des données sonores de l'expert (20) à l'utilisateur (10) et des données sonores de l'utilisateur (10) à l'expert (20).

8. Procédé selon l'une quelconque des revendications précédentes, dans lequel la création (400) d'un modèle de l'au moins un objet commandable (60) comprend la création d'un modèle 2D (80) de l'au moins un objet commandable (60), et/ou dans lequel la création (400) d'un modèle 2D de l'au moins un objet commandable comprend l'identification de points caractéristiques, de préférence d'articulations, de l'objet commandable, et la création d'une représentation schématique 2D de l'objet commandable sur la base des points caractéristiques identifiés, de préférence des articulations.

9. Support de stockage destiné à stocker des instructions d'un programme qui, lorsqu'il est exécuté sur un processeur, amène les étapes de l'une quelconque des revendications de procédé ci-dessus à être effectuées.

10. Dispositif électronique utilisateur (30) permettant l'assistance à distance d'un utilisateur (10) par un expert (20) ayant un dispositif expert (40) pour effectuer une tâche dans un environnement de travail (15), ledit dispositif utilisateur (30) étant associé à l'utilisateur (10), et comprenant :
- au moins une caméra (35) pour obtenir des données d'image de l'environnement de travail (15),
- des moyens de communication (36) pour communiquer les données d'image (50) de l'environnement de travail (15) au dispositif expert (40) et pour recevoir une scène de réalité augmentée, RA, (70) à partir du dispositif expert (40),
- un écran (31) pour afficher la scène de réalité augmentée, RA, reçue (70),
- un processeur (37) configuré pour effectuer au moins une ou plusieurs étapes selon l'une quelconque des revendications de procédé décrites ci-dessus.

11. Dispositif électronique utilisateur selon la revendication précédente, dans lequel le processeur (37) est configuré pour afficher la scène de réalité augmentée, RA, (70) dans une première fenêtre (33) de l'écran (31), dans lequel de préférence le processeur (37) est configuré pour afficher des données d'image de l'expert (20) dans une seconde fenêtre (34) de l'écran, et/ou le dispositif étant l'un quelconque parmi : une tablette, un téléphone intelligent, un ordinateur portable, un ou plusieurs visiocasques.

12. Dispositif électronique expert (40) permettant l'assistance à distance d'un utilisateur (10) ayant un dispositif utilisateur (30) par un expert (20) pour effectuer une tâche dans un environnement de travail (15), ledit dispositif expert étant associé à l'expert, et comprenant
- au moins une caméra (45), dans lequel la caméra (45) est configurée pour obtenir des informations de scène expert, les informations de scène expert comprenant des données d'image d'au moins un objet commandable par l'expert pour montrer comment effectuer la tâche,
- un processeur configuré pour effectuer au moins une ou plusieurs étapes selon l'une quelconque des revendications de procédé ci-dessus, et au moins configuré pour :
créer un modèle de l'au moins un objet commandable à partir des informations de scène expert obtenues,
générer une scène de réalité augmentée, RA, en incluant le modèle créé de l'au moins un objet commandable dans des données d'image de l'environnement de travail (15),
- des moyens de communication (46) pour recevoir des informations de scène utilisateur à partir du dispositif utilisateur comprenant des données d'image de l'environnement de travail (15), et pour communiquer au dispositif utilisateur la scène de réalité augmentée, RA,
- un écran (41) conçu pour afficher les informations de scène utilisateur sur le dispositif expert, et la scène de réalité augmentée, RA.

13. Dispositif électronique expert selon la revendication précédente, dans lequel la caméra et l'écran sont disposés sur le même côté du dispositif expert, et de préférence à proximité immédiate, et/ou dans lequel le dispositif comprend une seule caméra.

14. Dispositif électronique expert selon l'une quelconque des revendications 12 à 13, dans lequel le processeur (47) est configuré pour afficher la scène de réalité augmentée, RA, (70) dans une première fenêtre (43) de l'écran (41), dans lequel, de préférence, le processeur (37) est configuré pour afficher des données d'image de l'utilisateur (10) dans une seconde fenêtre (44) de l'écran (41), et/ou le dispositif étant l'un quelconque parmi : une tablette, un téléphone intelligent, un ordinateur portable.

15. Système d'assistance comprenant un dispositif électronique utilisateur selon l'une quelconque des revendications 10 à 11 et un dispositif électronique expert selon l'une quelconque des revendications 12 à 14.
